(19) Europäisches Patentamt
European Patent Office
Office européen des brevets

(11) **EP 1 101 100 B1**

(12) **FASCICULE DE BREVET EUROPEEN**

(45) Date de publication et mention
de la délivrance du brevet:
**16.06.2010 Bulletin 2010/24**

(51) Int Cl.:
**G01N 25/32** (2006.01)

(21) Numéro de dépôt: **00931358.6**

(86) Numéro de dépôt international:
**PCT/FR2000/001431**

(22) Date de dépôt: **26.05.2000**

(87) Numéro de publication internationale:
**WO 2000/073775 (07.12.2000 Gazette 2000/49)**

(54) **DISPOSITIF DE MESURE DE LA CONCENTRATION EN HYDROGENE DANS UN MELANGE GAZEUX**

VORRICHTUNG ZUR MESSUNG DES WASSERSTOFFGEHALTES IN EINEM GASGEMISCH

DEVICE FOR MEASURING THE CONCENTRATION OF HYDROGEN IN A GAS MIXTURE

(84) Etats contractants désignés:
**BE DE GB**

(30) Priorité: **28.05.1999 FR 9906782**

(43) Date de publication de la demande:
**23.05.2001 Bulletin 2001/21**

(73) Titulaire: **Commissariat à l'Energie Atomique
75752 Paris Cedex 15 (FR)**

(72) Inventeurs:
• **RONGIER, Pierre
F-83560 Vinon s/Verdon (FR)**
• **BONHOMME, Thierry
F-13410 Lambesc (FR)**
• **PEREZ, Christian
F-04180 Villeneuve (FR)**

(74) Mandataire: **Audier, Philippe André et al
Brevalex
3, rue du Docteur Lancereaux
75008 Paris (FR)**

(56) Documents cités:
**DE-A- 4 317 568    DE-A- 19 645 694
US-A- 4 298 574**

• **GARDNER W L: "SENSOR FOR MEASURING THE ATOMIC FRACTION IN HIGHLY DISSOCIATED HYDROGEN" JOURNAL OF VACUUM SCIENCE AND TECHNOLOGY: PART A,US,AMERICAN INSTITUTE OF PHYSICS. NEW YORK, vol. 13, no. 3, PART 01, 1 mai 1995 (1995-05-01), pages 763-766, XP000531584 ISSN: 0734-2101**
• **"improved O2/h2 gas-mixture sensor" NTIS TECH NOTES., vol. 7, no. J, 1984, pages 509-510, XP002133887 US DEPARTMENT OF COMMERCE. SPRINGFIELD, VA., US ISSN: 0889-8464**

**Description**

L'invention est relative à un dispositif de mesure de la concentration en hydrogène dans un mélange gazeux en particulier dans l'air constituant par exemple l'atmosphère d'un local fermé.

Le domaine technique de l'invention peut être défini comme celui de la mesure, c'est-à-dire de l'évaluation quantitative de la concentration ou taux d'hydrogène $H_2$ dans un mélange gazeux tel que.de l'air et de la vapeur d'eau ; cette atmosphère peut, en outre, être chargée en aérosols et poussière, suite à un accident.

Les dispositifs ou appareils permettant d'assurer une mesure qualitative ou quantitative de l'hydrogène dans un mélange gazeux peuvent en effet être classés, d'une part parmi les détecteurs, qui peuvent éventuellement être étalonnés en mesureurs dans une plage très limitée de teneurs, d'autre part parmi les appareils de laboratoire qui sont très complexes et qui nécessitent diverses alimentations en gaz de référence ainsi qu'un personnel spécialisé.

Il existe également des indicateurs qui ne sont pas actuellement utilisables dans une atmosphère accidentelle et dont la plage d'incertitudes est très large.

Les indicateurs les plus simples sont des catharomètres dont le principe est basé sur la mesure de la conductivité thermique du gaz contenu dans un local.

De tels appareils ne sont généralement utilisés que dans une atmosphère de type connue et ils sont donc spécifiquement étalonnés dans ce but. Ils nécessitent en outre une installation complexe avec un prélèvement de gaz, un condenseur, et des filtres.

Récemment, c'est-à-dire depuis environ cinq ans, s'est développée une très forte demande concernant des mesureurs pouvant être placés précisément au point où l'on souhaite connaître la teneur en hydrogène, et pouvant mesurer des teneurs en hydrogène dans une gamme de 0 à 30% de $H_2$, c'est-à-dire très au-delà des seuils d'inflammation, de déflagration et de détonation de l'hydrogène.

Ces demandes émanent principalement des exploitants mondiaux de centrales nucléaires. Un des risques majeurs en cas d'accident entraînant la fusion du coeur, est en effet le risque d'explosion dû à l'hydrogène, ce qui nécessite pour chaque réacteur la mise en oeuvre de plusieurs dizaines d'appareils.

Plusieurs types d'appareils mesureurs qui tentent de répondre aux demandes indiquées plus haut ont ainsi été mis au point et commercialisés. Ces mesureurs utilisent soit les caractéristiques électrochimiques, soit les caractéristiques thermiques de l'hydrogène.

Tous ces appareils ont l'inconvénient rédhibitoire de nécessiter une alimentation électrique permanente et importante, alors que, en cas d'accident grave, la première conséquence de celui-ci, est précisément, la perte de toute alimentation électrique.

Les présents inventeurs ont par ailleurs mis en évidence lors de tests concernant tous les mesureurs actuellement disponibles, qu'aucun de ceux-ci n'était acceptable, une des raisons fondamentales pour cela étant que l'atmosphère accidentelle dans le dôme d'une centrale nucléaire comporte une très grande proportion de vapeur d'eau et une très forte radioactivité, entre autres sous forme d'aérosols et de poussières, qui perturbent les systèmes de mesure.

Les appareils connus souffrent en outre d'un poids, d'un encombrement ainsi que d'un coût importants.

En conclusion, tous ces appareils quel que soit leur type présentent l'inconvénient d'un coût très élevé, d'une inadaptation à la demande exprimée et sont en définitive peu ou pas opérationnels.

US-A-4-298 574 décrit un dispositif de mesure de la concentration en hydrogène dans un mélange gazeux, comprenant un capteur apte à être mis en contact avec ledit mélange gazeux, dans lequel ledit capteur chauffé électriquement comprend un catalyseur susceptible de provoquer une réaction exothermique avec l'hydrogène contenu dans le mélange gazeux, un point froid, un fil en Chromel reliant ledit catalyseur et ledit point froid, et des moyens de mesure du gradient de température entre le catalyseur et le point froid.

I1 existe donc un besoin pour un dispositif de mesure de la concentration en hydrogène dans un mélange gazeux qui soit entre autres, simple, peu volumineux, d'un faible poids et qui présente une grande fiabilité.

Il existe encore un besoin pour un dispositif qui permette de mesurer avec précision les concentrations en hydrogène dans toutes les gammes de concentrations possibles, en particulier celles se trouvant au-delà des seuils d'inflammation, de déflagration et de détonation de l'hydrogène.

Ce dispositif doit pouvoir, en outre, être utilisé sans subir aucune perturbation pour mesurer la concentration en hydrogène de tout mélange gazeux quelle que soit sa composition, la présence éventuelle d'aérosols et de poussière, et sa radioactivité, comme c'est le cas, par exemple, de l'atmosphère régnant dans le dôme d'une centrale nucléaire suite à un accident.

Le but de la présente invention est de fournir un dispositif de mesure de la concentration en hydrogène dans un mélange gazeux qui réponde, entre autres à l'essentiel des besoins indiqués ci-dessus.

Le but de la présente invention est en outre de fournir un dispositif qui ne présente pas les inconvénients, limitations, défauts et désavantages des dispositifs de l'art antérieur et qui résolve les problèmes de l'art antérieur mentionnés ci-dessus.

Ce but est atteint, conformément à la présente invention, par un dispositif de mesure de la concentration en hydrogène dans un mélange gazeux comprenant un capteur en contact avec ledit mélange gazeux, tel que défini dans la revendication 1. Ledit capteur est relié à des moyens de calcul et d'affichage, et comprend un catalyseur susceptible de provoquer une réaction exothermique avec l'hydrogène contenu dans le mélange gazeux, des moyens conducteurs fixés audit catalyseur pour transférer essentiellement par conduction l'énergie thermique générée par ladite réaction depuis ledit catalyseur jusqu'à un point froid, des moyens de mesure de la température T1 dudit catalyseur et de la température T2 dudit point froid reliés aux moyens de calcul de la valeur de la concentration molaire en hydrogène dans le mélange gazeux à partir du gradient de température mesuré T1-T2, lesdits moyens de calcul fournissant ladite valeur de la concentration en hydrogène auxdits moyens d'affichage.

Le dispositif selon l'invention répond à tous les besoins indiqués plus haut, et apporte une solution aux problèmes de l'art antérieur.

De manière générale, le dispositif selon l'invention a une dimension ainsi qu'un poids qui sont très réduits par rapport aux dispositifs connus.

Aussi à titre d'exemple, le dispositif « capteur » selon l'invention pourra avoir des dimensions hors-tout de 10 à 15 cm et un poids généralement inférieur à 1 kilogramme.

Le faible poids et les faibles dimensions du dispositif selon l'invention permettent de manière particulièrement avantageuse de le positionner dans les endroits les plus difficiles d'accès, ce qui n'est pas le cas des appareils proposés actuellement.

De même, le dispositif selon l'invention est très simple, ne comporte qu'un nombre limité d'éléments, ce qui en réduit également le coût, en facilite la fabrication, la maintenance et contribue à sa fiabilité.

Le capteur du dispositif selon l'invention ne nécessite aucune alimentation électrique, au contraire des dispositifs de l'art antérieur, tel que le dispositif décrit dans le document US-A-4 298 574, qui font appel à une alimentation électrique permanente et importante.

En effet, le capteur ne comporte que des éléments qui génèrent eux-mêmes les énergies et tensions électriques utiles.

Cette autonomie du capteur le rend particulièrement adapté à sa mise en oeuvre dans les enceintes de centrale nucléaire à l'issue d'un accident puisqu'il peut rester opérationnel même si les systèmes d'alimentation électrique de la centrale font défaut.

Seuls les moyens de calcul et d'affichage, qui peuvent être déportés hors de la zone dangereuse, sont alimentés électriquement mais leur consommation reste très faible et leur alimentation peut être indépendante de l'alimentation générale de la centrale.

Ainsi, l'autonomie sur batterie du dispositif selon l'invention peut être 10 à 100 fois supérieure aux autonomies des dispositifs de l'art antérieur.

Par ailleurs, la nécessité d'alimentation et/ou de télécommande des pompes et des électrovannes des dispositifs de l'art antérieur accroît énormément la probabilité de non-fonctionnement très rapide d'un des éléments en séquence accidentelle.

En outre, selon l'invention, ces moyens de calcul et d'affichage de la concentration en $H_2$ peuvent être éloignés d'une distance quelconque du capteur, c'est-à-dire qu'ils peuvent se trouver par exemple à l'extérieur de l'atmosphère de la centrale et ne pas en subir les inconvénients lors d'une situation accidentelle.

Le capteur et les moyens de calcul et d'affichage sont reliés par tout moyen connu tel qu'un câble unique dont les dimensions sont faibles et la protection aux agressions très simplifiée.

L'invention repose, en particulier, sur l'utilisation d'une réaction exothermique de catalyse de l'hydrogène, par exemple de la réaction exothermique de catalyse de l'hydrogène avec l'oxygène de l'air contenu dans un mélange gazeux, pour transformer l'énergie produite par cette réaction en élément de mesure de la concentration en $H_2$ qui est, entre autres, simple, fiable et qui ne nécessite pas d'énergie électrique.

La mise en oeuvre de ces catalyseurs dans un tel but et le principe à la base de la mesure selon l'invention se démarque totalement de celui des dispositifs dans lesquels la mesure de la teneur en $H_2$ est essentiellement réalisée en suivant les variations des caractéristiques électrochimiques ou thermiques de l'hydrogène.

Il a été mis en évidence que le dispositif de l'invention, dans lequel le capteur comprend essentiellement un catalyseur du type décrit plus haut permettait des mesures fiables et précises, quelle que soit la composition du mélange gazeux, tel que de l'air et quelle que soit la concentration en hydrogène même élevée.

De manière particulièrement avantageuse, au contraire des dispositifs comportant un capteur opérant sur un principe différent, le dispositif de l'invention n'est pas perturbé par les aérosols, poussières et par la vapeur d'eau se trouvant dans le mélange gazeux, ainsi que par la radioactivité de celui-ci.

En effet, les catalyseurs du capteur selon l'invention ne sont pas « empoisonnés » par les aérosols et poussières, et la vapeur d'eau souvent en grande quantité qui leur est associée ne perturbe pas leur fonctionnement, de même que la radioactivité.

Le dispositif de mesure selon l'invention - du fait de sa caractéristique principale qui est l'utilisation d'un catalyseur - surmonte ainsi l'essentiel des problèmes rencontrés dans les dispositifs de l'art antérieur, notamment dans le cas des dispositifs utilisés dans l'atmosphère accidentelle des centrales nucléaires.

Le catalyseur qui est mis en oeuvre dans le capteur du dispositif selon l'invention est choisi de préférence parmi les catalyseurs dits catalyseurs de "recombinaison", c'est-à-dire les catalyseurs qui provoquent la recombinaison de l'hydrogène et de l'oxygène en $H_2O$.

Ledit catalyseur de recombinaison est choisi de préférence parmi le platine et le palladium.

C'est l'énergie exothermique considérable créée par la réaction avec le catalyseur, par exemple par la réaction de recombinaison, qui conduit à une élévation de température du catalyseur (jusqu'à la température T1), et le gradient thermique T1 - T2 (où T2 est généralement voisin de la température ambiante), peut être utilisé pour la mesure de la concentration en hydrogène.

Le dispositif selon l'invention fournit en outre une gestion adaptée des gradients thermiques créés par le phénomène exothermique décrit ci-dessus, qui empêche les catalyseurs de devenir igniteurs de l'inflammation de l'hydrogène, comme c'est le cas pour les catalyseurs "recombineurs".

Selon l'invention, l'énergie thermique apportée par la réaction catalytique est canalisée vers un point froid, d'une part de façon à éviter que la température ne montre trop haut, et à écarter tout risque d'ignition du mélange air et hydrogène, et, d'autre part, de façon à disposer d'un paramètre T1-T2 (T1 représentant la température du catalyseur consécutivement à la réaction exothermique et T2 représentant la température du point froid) proportionnel à l'énergie dégagée.

Selon l'invention, ledit point froid vers lequel est canalisée l'énergie de la catalyse est constitué d'une ailette de refroidissement, dont la température est généralement de l'ordre de la température ambiante.

Par ailleurs, avantageusement, le capteur selon l'invention peut être placé dans une canalisation ou cheminée de laquelle il est solidaire et dans laquelle circule le mélange gazeux. Le capteur, dans le cas où la mesure s'effectue dans une atmosphère agitée, par exemple l'atmosphère accidentelle d'une centrale nucléaire, est ainsi protégé des turbulences aérodynamiques, des hétérogénéités locales et d'une éventuelle aspersion d'eau directe sur le catalyseur.

Le capteur établit dans la canalisation ou cheminée, généralement tubulaire, une convection qu'il contrôle luimême en se comportant comme une sorte de pompe.

De préférence, ladite canalisation ou cheminée-est de faible dimension, c'est-à-dire qu'elle se présente, par exemple sous la forme d'un tube ou cylindre d'une longueur de 10 à 15 cm et d'un diamètre de 2 à 3 cm. Cette canalisation peut également présenter une section rectangulaire de dimensions, par exemple 1,5 x 3 cm, avec une longueur, par exemple de 10 à 15 cm. La canalisation ou cheminée ne participe donc que faiblement à l'encombrement du capteur qui peut être placé dans des endroits habituellement inaccessibles aux autres capteurs.

Selon l'invention, le transfert d'énergie entre le point chaud (catalyseur à la température T1) du capteur et le point froid (ailette de refroidissement à la température T2) se fait essentiellement par conduction.

Selon la formule (1) des échanges thermiques par conduction donnée ci-dessous, la relation entre le gradient thermique T1-T2 et l'énergie dégagée est linéaire :

$$P(W) = ((\lambda * S) / l) * (T1 - T2) \qquad (1)$$

où :

- l est la longueur du conducteur en cm ;
- S est la section du conducteur en $cm^2$ ;
- $\lambda$ est la conductivité du matériau en $W.cm^{-1}.c^{-1}$.

De préférence, comme indiqué ci-dessus, la cheminée qui est la partie la plus encombrante du dispositif est verticale et de section rectangulaire.

La partie « capteur » est généralement perpendiculaire à la cheminée et est généralement de forme extérieure tubulaire, cylindrique.

Les moyens conducteurs se présentent généralement également sous la forme d'un tube ou cylindre.

Selon l'invention, pour favoriser les échanges thermiques par conduction, on réduit de manière avantageuse les échanges thermiques par rayonnement et par convection, qui eux, ne sont pas linéaires.

Ainsi, selon l'invention et afin de réduire les échanges thermiques par convection, lesdits moyens conducteurs sont entourés par l'enveloppe du capteur et séparés de celle-ci par une épaisseur de gaz, tel que de l'air, suffisamment faible, par exemple de 2 à 3 mm, pour qu'il n'y ait pas d'échanges thermiques par convection dans cet espace ainsi défini entre les moyens conducteurs et l'enveloppe.

De même, selon l'invention, la surface du catalyseur est telle que l'énergie catalytique (et donc la température du conducteur) est suffisamment faible pour rendre négligeables les échanges thermiques par rayonnement (par rapport aux échanges thermiques par conduction). La surface du catalyseur est donc une surface que l'on peut qualifier de faible, c'est-à-dire voisine, par exemple de 1 cm$^2$.

Le dispositif selon l'invention comprend également des moyens de mesure de la température T1 du catalyseur (point chaud), et de la température T2 du point froid. Ces moyens sont généralement constitués par des thermocouples, qui sont généralement connectés tête-bêche et permettent de disposer directement et sans soudure froide, d'une mesure proportionnelle du gradient thermique.

Le dispositif selon l'invention comprend des moyens de calcul et d'affichage de la concentration molaire en hydrogène qui sont reliés auxdits moyens de mesure de la température.

Dans ces moyens de calcul, on réalise le calcul de la concentration molaire en hydrogène à partir des mesures des températures T1 et T2.

Le gradient de température (T1-T2) est en effet l'image de la pression partielle d'hydrogène.

Par définition, la concentration molaire en $H_2$ est donnée par la formule :

$$C(H_2) \text{molaire} = ((\text{Pression partielle } H_2)/(\text{Pression totale atmosphère ambiante}))*100 \qquad (2)$$

que nous écrirons :

$$C(H_2) = (P(H_2)/Pt)*100 \qquad (3)$$

La pression totale Pt est égale à la somme de la pression atmosphérique (Pa), de la pression partielle de vapeur d'eau (P($H_2O$)), et de la pression partielle d'hydrogène (P($H_2$)) selon l'équation :

$$Pt = Pa + P(H_2O) + P(H_2) \qquad (4)$$

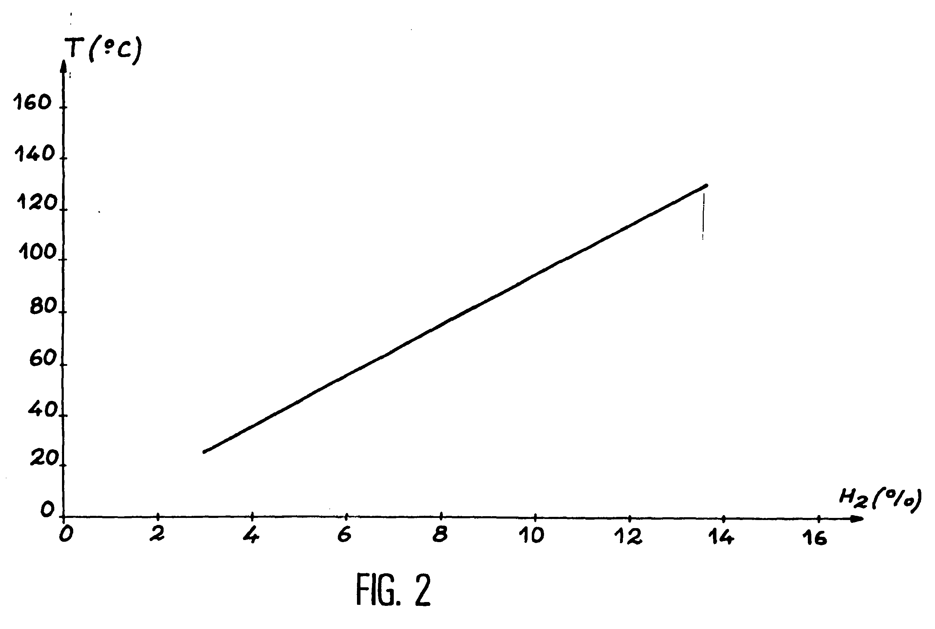

dans laquelle Pa = 1 ; et P($H_2$) est donnée par la mesure de (T1-T2) par l'intermédiaire d'une fonction de transfert qui est une droite (voir par exemple la figure 2).

Pour chaque capteur, il est généralement nécessaire d'effectuer un étalonnage préalable pour obtenir cette fonction de transfert ou courbe d'étalonnage, c'est-à-dire que chaque capteur est ainsi associé à sa courbe d'étalonnage.

Toutefois, cette courbe est la même pour chaque type de catalyseur. Il sera donc intéressant de ne retenir qu'un seul type de catalyseur. Dans ce cas, un stock de plaques catalytiques de fabrication unique sera suffisant pour plusieurs années de fourniture.

Cette fonction de transfert est introduite dans les moyens de calcul.

Dans certaines applications, la pression partielle de vapeur d'eau P$H_2O$ peut être négligée, si bien que les deux moyens de mesure des températures T1 et T2 sont suffisants pour connaître P($H_2$) et Pt.

Par exemple dans le cas d'une centrale nucléaire, l'atmosphère ambiante, hors accident, est de l'air à la pression atmosphérique.

La dépression, qui est de l'ordre du millibar peut être négligée, et il en est de même de la pression partielle de vapeur d'eau qui est de l'ordre de 60% de la saturation à 25°C, soit inférieure à 10 mbar.

On peut donc assimiler l'atmosphère d'origine à de l'air sec à 1 bar.

Avantageusement, selon l'invention, dans le cas où la pression partielle de vapeur d'eau ne peut être négligée, il est alors nécessaire de prévoir en outre des moyens de mesure de la température ambiante T3, qui peuvent également prendre la forme, par exemple, d'un thermocouple et qui sont reliés auxdits dispositifs de calcul et d'affichage.

Grâce à cette mesure de T3, on peut déterminer la pression partielle P($H_2O$) à l'aide des tables de pression saturante, tandis que P($H_2$) est déterminé comme indiqué ci-dessus et que Pa = 1 bar, on en déduit facilement Pt dans l'équation (4) et donc C($H_2$) dans l'équation (3).

Les tables donnant P($H_2O$) en fonction de T3 sont également introduites dans les moyens de calcul.

Par exemple, de nouveau dans le cas d'une centrale nucléaire en situation accidentelle, l'atmosphère de la centrale se charge très rapidement en vapeur d'eau, et l'énergie de celle-ci est telle qu'elle impose une pression partielle de $H_2O$ égale à la pression saturante à la nouvelle température ambiante.

Dans le même temps, il y a génération d'hydrogène incondensable dont la pression partielle vient s'ajouter aux deux pressions précédentes (car pour mémoire, la détection d'une situation accidentelle provoque l'isolement étanche de la centrale vis-à-vis de l'extérieur), et Pt est déterminé par l'équation (4) ci-dessus, dans laquelle :

- P($H_2$) est donné par la courbe de réponse du capteur par mesure de T1-T2 ;
- P($H_2O$) est donnée par les tables de pression saturante, par mesure de T3 ;
- P(air) est prise égale à 1 bar.

Dans d'autres utilisations du dispositif selon l'invention, c'est à l'utilisateur de définir l'atmosphère d'origine, et de manière avantageuse, selon l'invention, la pression totale Pt du mélange gazeux est alors éventuellement mesurée par un capteur de pression.

Ceci inclut par exemple le cas où l'exploitant ne souhaite pas négliger la pression partielle des autres gaz incondensables non pris en compte.

Les caractéristiques et avantages de l'invention apparaîtront mieux à la lecture de la description qui va suivre. Cette description, donnée à titre illustratif et non limitatif, est faite en référence aux dessins joints, dans lesquels :

- la figure 1 représente une vue schématique en coupe transversale d'un dispositif de mesure selon l'invention ;

- la figure 2 est une courbe qui donne la température T1-T2 (en °C) en fonction de la pression partielle de $H_2$ (%).

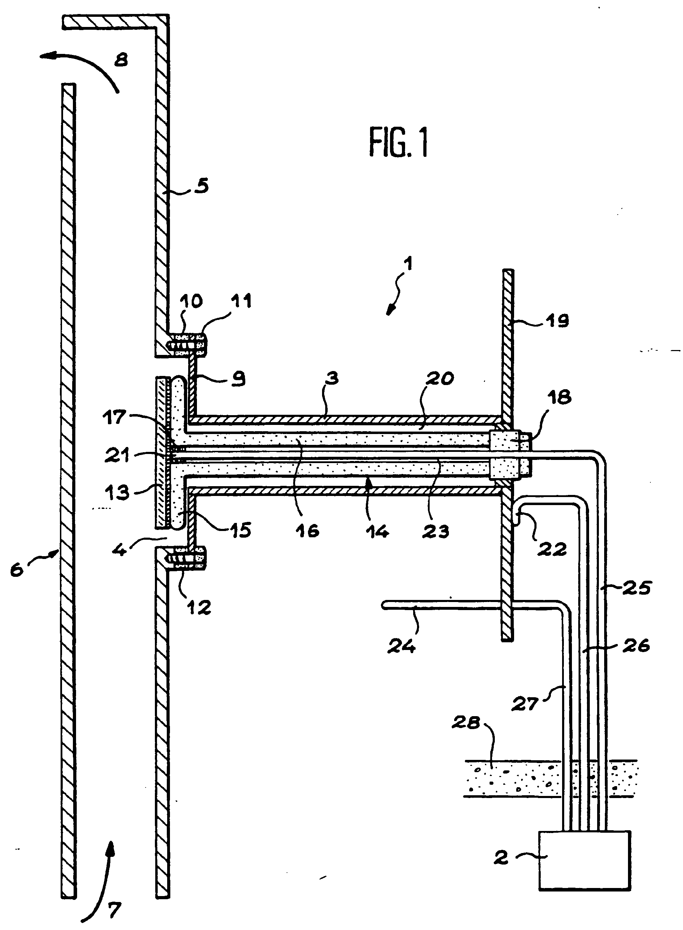

Sur la figure 1, on a ainsi représenté un dispositif de mesure selon l'invention, ce dispositif comprend essentiellement un capteur (1) relié à un dispositif de calcul et d'affichage (2).

Le capteur représenté sur la figure 1 présente une forme sensiblement tubulaire avec une enveloppe (3) extérieure constituée d'un tube en un matériau tel que de l'acier inoxydable. Le capteur par l'intermédiaire de cette enveloppe extérieure est fixé dans un orifice ou perçage (4) prévu dans la paroi latérale (5) d'une canalisation ou cheminée (6) qui permet de protéger le capteur notamment des turbulences aérodynamiques, des hétérogénéités locales et d'une éventuelle aspersion d'eau directe sur l'organe sensible du dispositif.

Cette cheminée, qui a généralement la forme d'une canalisation de section rectangulaire, est de faible dimension : par exemple d'une longueur de 10 à 15 cm et d'une section de 1,5 à 3 cm.

Le dispositif selon l'invention établit dans cette cheminée (6), par son propre fonctionnement, une convection qu'il contrôle lui-même en se comportant comme une sorte de pompe.

Ainsi, sur la figure 1, le mélange gazeux pénètre-il en (7) dans la cheminée, et ressort en (8) de celle-ci.

La cheminée est généralement réalisée en un matériau tel que l'acier inoxydable.

La fixation de l'enveloppe du capteur à la paroi de la cheminée est réalisée par l'intermédiaire d'une partie formant bride (9), de filetages (10) et d'écrous (11), un isolant thermique (12) étant intercalé entre ladite enveloppe (3) et ladite paroi (5).

Il est à noter que cet isolant thermique peut être éventuellement supprimé, la cheminée jouant alors le rôle d'ailette de refroidissement.

Cet isolant thermique est, par exemple en Téflon® et se présente sous la forme d'un anneau, par exemple de diamètre intérieur 2 cm, d'épaisseur 0,5 cm et de largeur 1 cm.

Le capteur comporte, en tant qu'élément essentiel, un catalyseur (13) qui, sur la figure 1, a la forme d'un disque, par exemple, d'un diamètre de 12 mm et d'une épaisseur de 0,1 à 0,2 mm.

Le catalyseur pourrait également prendre une autre forme, par exemple celle d'un carré de côté, par exemple de 12 mm.

Une telle taille du catalyseur permet de réduire au minimum, les échanges thermiques par rayonnement. Le catalyseur se trouve placé dans le perçage ou orifice de la paroi de la cheminée, de façon à se trouver en contact avec le courant de mélange gazeux en circulation dans celle-ci.

Le catalyseur est généralement en Pt et/ou en Pd généralement sur un support inerte d'oxyde $Al_2O_3$ ou d'un autre oxyde.

Le catalyseur est fixé à un élément conducteur (14), comprenant généralement une première partie formant support de catalyseur, généralement en forme de disque (15) de même diamètre que le catalyseur, ladite première partie étant raccordée à une seconde partie (16) de l'élément, généralement en forme de tube allongé, d'une longueur de 2 à 4 cm, et de diamètre plus faible que celui de la première partie généralement en forme de disque, c'est-à-dire de 4 à 5 mm.

L'élément conducteur est généralement réalisé en inox.

La fixation du catalyseur sur ladite première partie du conducteur est généralement réalisée par brasage (17) ; mais une fixation totalement mécanique peut être envisagée pour éviter cette opération délicate.

L'extrémité (18) dudit tube conducteur opposée à l'extrémité supportant le catalyseur constitue le « point froid » du capteur selon l'invention.

Sur la figure 1, ce point froid est maintenu à-une température très proche de l'ambiante par une ailette de refroidissement (19) fixée à ladite extrémité (18) du tube conducteur et raccordée à l'enveloppe du capteur.

Les dimensions de ladite ailette sont, par exemple de 4 à 10 cm et sont telles que sa température T2 ne soit pas supérieure à la température ambiante de plus de 5°C, cette température doit être suffisamment faible pour qu'il ne se produire pas d'élévation trop importante de température au niveau du catalyseur et du conducteur et que la chaleur de la réaction exothermique soit dissipée. La longueur du conducteur qui est de 2 à 4 cm est également choisie dans ce but.

En outre, et afin de réduire, voire d'annuler les échanges par convection, l'espace entre l'élément conducteur (14), celui-ci est séparé de l'enveloppe (3) du capteur par une épaisseur de gaz (20), tel que de l'air qui doit être la plus faible possible, par exemple de 2 à 3 mm.

Le dispositif selon l'invention comprend ensuite des moyens de mesure de la température, du catalyseur T1 (21), et de la température T2 (22) du point froid, c'est-à-dire de la température de l'ailette de refroidissement.

Les moyens sont constitués, sur la figure 1, par des thermocouples (21, 22) de type K d'un diamètre de 1 mm, respectivement prévus au voisinage du catalyseur (13) et de l'ailette de refroidissement (19).

Le thermocouple de mesure de la température T1 du catalyseur est placé dans un alésage (23) de l'élément conducteur en forme de tube au voisinage immédiat du catalyseur, par exemple, sous le disque du catalyseur (13) et contre la brasure (17).

En outre, le dispositif, représenté sur la figure 1, comporte un troisième thermocouple (24) de mesure de la température ambiante T3.

Les thermocouples (21, 22, 24) sont reliés par l'intermédiaire de câbles (25, 26, 27) au dispositif de calcul et d'affichage (2). Les trois câbles (25, 26, 27) peuvent être regroupés pour ne plus en faire qu'un seul d'un diamètre faible, par exemple, inférieur à 10 mm.

La longueur dudit câble n'est pas limitée, si bien que les dispositifs d'affichage et de calcul peuvent se trouver en un point éloigné du capteur, par exemple, en un point extérieur à l'enceinte (28) dans laquelle est effectuée la mesure.

Les dispositifs d'affichage et de mesure (2) ne sont donc pas exposés à l'atmosphère agressive à laquelle est soumise le capteur, par exemple dans l'enceinte d'une centrale nucléaire lors d'un accident, et leur fonctionnement peut être autonome, ne dépend pas de l'alimentation générale de ladite centrale.

Les dispositifs d'affichage et de calcul sont des dispositifs classiques, de faible dimension et de faible coût. Leur alimentation peut être faite par une tension de 220 V alternative, ou par une tension de 24 V continue pour une consommation réduite, de l'ordre de 20 W.

Le dispositif de calcul comprend, en particulier, une mémoire dans laquelle sont stockées les tables de pression de vapeur d'eau saturante, qui permettent de calculer $P(H_2O)$ par introduction de la valeur T3 de la température ambiante. Dans ces moyens de mémoire est également stockée la courbe qui donne la pression partielle de $H_2$ en fonction du gradient de température T1 - T2.

Une telle courbe est généralement une droite, par exemple, d'équation $y = 9,9754 x - 4,736$, comme c'est le cas sur la figure 2.

En introduisant les valeurs T1 et T2 depuis les moyens de mesure dans le dispositif de calcul, celui-ci calcule T1 - T2, puis $P(H_2)$, grâce à ladite droite.

Le dispositif de calcul (2) nécessite donc un étalonnage préalable par type de capteur lui permettant de calculer ladite fonction de transfert ou droite qui est spécifique pour chaque type de capteur, et qui est ensuite stockée dans la mémoire dudit dispositif de calcul.

Le dispositif de calcul déduit ensuite la valeur de $C(H_2)$, cette valeur est fournie au dispositif d'affichage, tel qu'un écran... ou autre pour visualiser, un dispositif d'alarme sonore ou lumineux peut être également prévu.

**Revendications**

1. Dispositif de mesure de la concentration en hydrogène dans un mélange gazeux comprenant un capteur (1) apte à être mis en contact avec ledit mélange gazeux, et des moyens de calcul et d'affichage (2) de la concentration molaire en hydrogène dans le mélange gazeux reliés audit capteur, dans lequel ledit capteur comprend un catalyseur (13) susceptible de provoquer une réaction exothermique avec l'hydrogène contenu dans le mélange gazeux, un point froid (18) constitué par une ailette de refroidissement (19), des moyens conducteurs (14) fixés audit catalyseur pour transférer essentiellement par conduction l'énergie thermique générée par ladite réaction depuis ledit catalyseur (13) jusqu'audit point froid (18), et des moyens de mesure de la température T1 (21) dudit catalyseur et de la température T2 (22) dudit point froid (18) reliés auxdits moyens de calcul, lesdits moyens de calcul calculant la valeur de la concentration molaire en hydrogène dans le mélange gazeux, à partir du gradient de température mesuré T1-T2, et fournissant ladite valeur de la concentration en hydrogène auxdits moyens d'affichage ; la surface du catalyseur (13) étant en outre suffisamment faible pour rendre négligeables les échanges thermiques par rayonnement par rapport aux échanges thermiques par conduction ; et le capteur comprenant, en outre, une enveloppe extérieure (3) et lesdits moyens conducteurs étant entourés par l'enveloppe (3) du capteur et séparés de celle-ci par une épaisseur (20), cette épaisseur étant suffisamment faible pour que, lorsqu'elle est remplie de gaz, il n'y ait pas d'échanges thermiques par convection dans l'espace défini entre l'enveloppe et les moyens conducteurs.

**2.** Dispositif selon la revendication 1, dans lequel ledit catalyseur (13) est choisi parmi les catalyseurs de recombinaison de l'oxygène et de l'hydrogène.

**3.** Dispositif selon la revendication 2, dans lequel ledit catalyseur (13) est choisi parmi le platine et le palladium.

**4.** Dispositif selon l'une quelconque des revendications 1 à 3, qui comprend en outre une canalisation ou cheminée dans laquelle est placée le capteur et de laquelle il est solidaire, ladite canalisation ou cheminée étant apte à recevoir une circulation du mélange gazeux.

**5.** Dispositif selon l'une quelconque des revendications 1 à 4, comprenant, en outre, des moyens de mesure de la température ambiante T3.

**6.** Dispositif selon la revendication 5, dans lequel les moyens de mesure de la température ambiante T3 sont constitués par un thermocouple (24).

**7.** Dispositif selon l'une quelconque des revendications 1 à 6, comprenant, en outre, un capteur de pression pour mesurer la pression totale Pt du mélange gazeux.

**8.** Dispositif selon l'une quelconque des revendications 1 à 7, dans lequel lesdits moyens de mesure des températures T1 et T2 sont constitués par des thermocouples (21, 22).

**9.** Dispositif selon la revendication 8, dans lequel lesdits thermocouples de mesure de la température T1 du catalyseur (13) et de la température T2 du point froid (18) sont connectés tête-bêche et sans soudure froide.

**Claims**

**1.** Device for measuring the hydrogen concentration in a gaseous mixture comprising a sensor (1) suitable for being placed in contact with said gaseous mixture, and means for calculating and displaying (2) the molar hydrogen concentration in the gaseous mixture connected to said sensor, wherein said sensor comprises a catalyst (13) suitable for inducing an exothermal reaction with the hydrogen contained in the gaseous mixture, a cold point (18) consisting of a cooling fin (19), conducting means (14) attached to said catalyst to transfer essentially by conduction the heat energy generated by said reaction from said catalyst (13) to said cold point (18), and means for measuring the temperature T1 (21) of said catalyst and the temperature T2 (22) of said cold point (18) connected to said calculation means, said calculation means calculating the molar hydrogen concentration in the gaseous mixture, on the basis of the temperature gradient measured T1-T2, and supplying said hydrogen concentration values to said display means; the surface area of the catalyst (13) furthermore being sufficiently small to render the heat exchanges by radiation negligible with respect to the heat exchanges by conduction; and the sensor further comprising an external casing (3) and said conducting means being encompassed by the casing (3) of the sensor and separated from same by a thickness (20), said thickness being sufficiently small such that, when filled with gas, no heat exchange by convection occurs in the space defined between the casing and the conducting means.

**2.** Device according to claim 1, wherein said catalyst (13) is selected from oxygen and hydrogen recombination catalysts.

**3.** Device according to claim 2, wherein said catalyst (13) is selected from platinum and palladium.

**4.** Device according to any of claims 1 to 3, further comprising a duct or shaft wherein the sensor is placed and whereto it is rigidly connected, said duct or shaft being suitable for receiving a gaseous mixture flow.

**5.** Device according to any of claims 1 to 4, further comprising means for measuring the ambient temperature T3.

**6.** Device according to claim 5, wherein the means for measuring the ambient temperature T3 consist of a thermocouple (24).

**7.** Device according to any of claims 1 to 6, further comprising a pressure sensor for measuring the total pressure Pt of the gaseous mixture.

**8.** Device according to any of claims 1 to 7, wherein said means for measuring temperatures T1 and T2 consist of thermocouples (21, 22).

9. Device according to claim 8, wherein said thermocouples for measuring temperature T1 of the catalyst (13) and temperature T2 of the cold point (18) are connected head-to-tail and without a cold junction.

**Patentansprüche**

1. Vorrichtung zur Messung der Wasserstoffkonzentration in einem Gasgemisch, umfassend einen zur Kontaktherstellung mit dem genannten Gasgemisch fähigen Sensor (1) und mit dem genannten Sensor verbundene Einrichtungen (2) zur Berechnung und Anzeige der molaren Wasserstoffkonzentration in dem Gasgemisch, bei der der genannte Sensor umfasst: einen eine exothermische Reaktion mit dem in dem Gasgemisch enthaltenen Wasserstoff bewirkenden Katalysator (13), einen durch eine Kühlrippe (19) gebildeten kalten Punkt (18), an dem genannten Katalysator befestigte Leiteinrichtungen (14), um die durch die genannte Reaktion erzeugte Wärme im Wesentlichen durch Leitung von dem genannten Katalysator (13) zu dem kalten Punkt (18) zu übertragen, und Messeinrichtungen der Temperatur T1 (21) des genannten Katalysators und der Temperatur T2 (22) des genannten kalten Punkts (18), verbunden mit den genannten Berechnungseinrichtungen, wobei die genannten Berechnungseinrichtungen aufgrund des gemessenen T1-T2-Temperaturgradienten den Wert der molaren Wasserstoffkonzentration in dem Gasgemisch berechnen und den Anzeigeeinrichtungen den genannten Wert der Wasserstoffkonzentration liefern; und die Oberfläche des Katalysators (13) außerdem hinreichend klein ist, um die Wärmeaustausche durch Strahlung vernachlässigbar zu machen in Bezug auf die Wärmeaustausche durch Leitung; und der Sensor außerdem eine Außenhülle (3) umfasst und die genannten Leiteinrichtungen von der Hülle (3) des Sensors umgeben und von dieser durch eine Dicke (20) getrennt sind, wobei diese Dicke so klein ist, dass es, wenn sie mit Gas gefüllt ist, in dem Raum zwischen der Hülle und den Leiteinrichtungen keine Wärmeaustausche durch Konvektion gibt.

2. Vorrichtung nach Anspruch 1, bei der der genannte Katalysator (13) ausgewählt wird unter den Wasserstoff- und Sauerstoff-Rekombinationskatalysatoren.

3. Vorrichtung nach Anspruch 2, bei der der genannte Katalysator ausgewählt wird zwischen Platin und Palladium.

4. Vorrichtung nach einem der Ansprüche 1 bis 3, die außerdem einen Kanal oder Kamin umfasst, in dem sich der Sensor befindet und mit dem er fest verbunden ist, wobei dieser Kanal oder Kamin für eine Zirkulation des Gasgemisches geeignet ist.

5. Vorrichtung nach einem der Ansprüche 1 bis 4, die außerdem Messeinrichtungen der Umgebungstemperatur T3 umfasst.

6. Vorrichtung nach Anspruch 5, bei der die Messeinrichtungen der Umgebungstemperatur T3 durch ein Thermoelement (24) gebildet werden.

7. Vorrichtung nach einem der Ansprüche 1 bis 6, die außerdem einen Drucksensor zur Messung des Gesamtdrucks Pt des Gasgemisches umfasst.

8. Vorrichtung nach einem der Ansprüche 1 bis 7, bei der die genannten Messeinrichtungen der Temperaturen T1 und T2 durch Thermoelemente (21, 22) gebildet werden.

9. Vorrichtung nach Anspruch 8, bei der die genannten Thermoelemente zur Messung der Temperatur T1 des Katalysators (13) und der Temperatur T2 des kalten Punkts (18) entgegengesetzt und ohne Kaltlötung verbunden sind.

# FIG. 1

FIG. 2

EP 1 101 100 B1

**EP 1 101 100 B1**